# FASCICULE DE BREVET EUROPEEN

(11) **EP 1 453 916 B1**
(45) Date de publication et mention de la délivrance du brevet: **11.05.2011**
(21) Numéro de dépôt: 02804612.6
(22) Date de dépôt: 12.12.2002
(51) Int. Cl.: C08L 5/08, A61K 9/00

(54) **ASSOCIATION CONTENANT UN POLOXAMER ET DE L'ACIDE CHONDROITINE SULFURIQUE ET/OU UNE GLYCOPROTÉINE ET SON UTILISATION**
KOMPLEX VON POLYOXAMER UND CHONDROITINSULFAT UND/ODER GLYCOPROTEIN UND SEINE VERWENDUNG
COMBINATION CONTAINING A POLOXAMER AND CHONDROITIN SULPHURIC ACID AND/OR A GLYCOPROTEIN AND USE THEREOF

(30) Priorité: 12.12.2001 FR 0116033
(43) Date de publication de la demande: 08.09.2004
(73) Titulaire: Pierre Fabre Dermo-Cosmétique, 92100 Boulogne-Billancourt (FR)
(72) Inventeur: MAVON, Alain, F-31450 Corronsac (FR); BORDAT, Pascal, F-31320 Mervilla (FR); COUSSE, Henri, F-81100 Castres (FR)
(74) Mandataire: Ahner, Francis
(86) Numéro de dépôt international: PCT/FR2002/004313
(87) Numéro de publication internationale: WO 2003/050190

(56) Documents cités:
- WO-A-00/51619
- WO-A-91/16058
- US-A- 4 740 498
- US-A- 6 133 325
- US-B1- 6 316 428
- TOPCHIEVA I N: "COMPLEXATION BETWEEN SS-CYCLODEXTRINS AND POLY(ETHYLENE OXIDE)-POLY(PROPYLENE OXIDE) BLOCK COPOLYMERS" POLYMER SCIENCE, INTERPERIODICA, BIRMINGHAM, AL, US, vol. 35, no. 4, 1 avril 1993 (1993-04-01), pages 464-466, XP000364815 ISSN: 0965-545X

## Description

La présente invention concerne une nouvelle association pour son utilisation dans une formulation cosmétique hydratante d'un poloxamer et d'un polymère choisi parmi l'acide chondroïtine sulfurique (ACS), une glycoprotéine ou un mélange d'acide chondroïtine sulfurique et d'une glycoprotéine.

Le maintien d'une bonne hydratation de surface cutanée revêt une importance majeure dans la physiologie cutanée [Harding et al. Int J Soc Chem, 22 :21 (2000), review]. Cette hydratation est obtenue par la présence d'une organisation de lipides en phases lamellaires entourant les cornéocytes, qui a pour objet de bloquer la perte en eau [Elias et al. Sem in Dermatol, 11,2 :176, (1992)]. La fixation de l'eau, *in situ,* est assurée par des molécules hygroscopiques, auxquelles il faut ajouter, en surface, la présence d'un film hydrolipidique [Sheu et al. br J dermatol, 140 : 385, (1999)], formé d'eau et de lipides sécrétés par les glandes sébacées, essentiellement localisé sur le visage.

Consécutivement à des agressions externes (froid, vent, savon agressif) cette barrière peut perdre une partie de cette protection et donc devenir sèche. Des états pathologiques peuvent aussi être à l'origine d'une sécheresse cutanée telle que la dermatite atopique [Fartasch et al. Ȧcta Derm Venerol, 176 :26 (1992)]. La conséquence de cette sécheresse se traduit par une sensation d'inconfort et de tiraillement, et à long terme par une accélération du vieillissement cutané. Dans le cas de certaines pathologies s'ajoutent des irritations et des inflammations cutanées induisant alors une forte altération de la barrière cutanée.

Dans la physiologie humaine, l'oeil, tout comme la peau, est un organe ayant une très grande surface en contact avec le milieu extérieur. Cette surface de contact, c'est à dire la cornée, est une muqueuse qui doit être en permanence hydratée pour permettre une vision optimum (transparence) et pour maintenir son intégrité biologique. Cette "couverture" est appelée le film oculaire [Holly, in Contact angle, Wettability & Adhesion, Mittal Ed : 849 (1993)]. Il est composé de larmes qui assurent l'hydratation et la lubrification de la cornée ainsi que d'un film de lipides qui recouvre ce film de larmes afin de freiner l'évaporation de l'eau. Ce film de lipides, le meibum, est composé en majorité de cires et de phospholipides [Tiffany, Cell Biochem & Funct. 13 : 177 (1995)].

Dans les larmes, l'eau est associée à des sels et est surtout structurée par diverses protéines et glycoprotéines. Ces glycoprotéines, sont essentiellement des mucines [Tiffany et al. invest Ophtalmic Vis, Sci 37 : 845(1996)]. Elles modifient la tension de surface ce qui permet de faciliter l'étalement des larmes sur la cornée. La famille de protéines des lipocalines ou préalbumines [Glasgow et al. Curr Eye Res, 14 : 363 (1995)], sont impliquées dans la stabilisation du film de lipides (meibum) sur la phases aqueuse, afin de maintenir sa continuité. Ainsi le film oculaire est défini comme un film hydrolipidique agissant comme hydratant et comme protecteur de la cornée. Lorsque ce film fait défaut ou l'un de ces composants on parle de pathologies décrites sous le nom générique de "syndrome de l'oeil sec" [Hoang-Xuan et al. EMC, 6-0010 (1998)], caractérisé par une sécheresse oculaire qui conduit à des gênes importantes de la vision du fait d'une irritation de la cornée. Le traitement des pathologies du «syndrome de l'oeil sec» est possible par l'apport de larmes artificielles.

La demande de brevet WO9906022 divulgue une formulation mimant la composition chimique et les propriétés physicochimiques du film oculaire à base de lipides.

Par ailleurs, l'utilisation de glycoprotéines type mucines dans l'hydratation cutanée a été décrite dans la demande de brevet EP 149 872.

De plus, la demande de brevet WO9116058 décrit une composition sous forme de gel aqueux, pour traiter de manière topique des tissus malades ou abîmés, comprenant une solution de copolymère bloc polyoxyéthylène-polyoxyproylène et une quantité efficace de mucopolysaccharide acide, le copolymère étant le composant majoritaire de la composition.

Le brevet US 6 316 428 divulgue des compositions à base de lécithine pour traiter et hydrater, par application topique, des structures à base de kératine (peau, cheveux, ongles, etc.), ces compositions pouvant en outre contenir un poloxamer. Il est précisé que ces compositions peuvent permettre de véhiculer des molécules telles que le sulfate de chondroïtine mais sans indication des proportions des différents composants de la composition.

On a maintenant constaté de manière tout à fait surprenante et inattendue qu'une association de type hydrolipidique et mimant les conditions physicochimiques du film oculaire (assurant l'hydratation de la cornée) permettait d'améliorer l'hydratation cutanée et/ou de traiter la sécheresse cutanée.

La présente invention a ainsi pour objet une association pour son utilisation dans une formulation cosmétique hydratante d'un poloxamer et d'un polymère choisi parmi l'acide chondroïtine sulfurique, une glycoprotéine ou un mélange d'acide chondroïtine sulfurique et d'une glycoprotéines.

Dans le cadre de la présente invention, le « poloxamer » désigne le copolymère séquencé polyoxyethylène-polyoxypropylène-polyoxyethylène (POE-POP-POE) de formule générale :

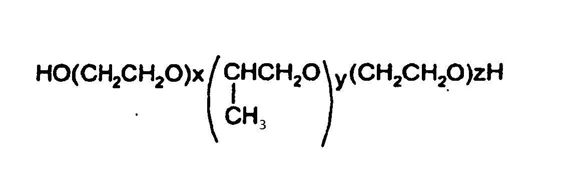

Dans cette formule générale, avantageusement, la valeur moyenne de x est comprise entre 2 et 128, la valeur moyenne de y est comprise entre 16 et 67 et la valeur moyenne de z est comprise ente 2 et 128.

On préfère le poloxamer 188 (avec n motif oxyethylène et m motif propylène : n = 75, m = 30 et n = 75) ou le poloxamer 407 (avec n motif oxyethylène et m motif propylène : n = 98, m = 67, n = 98) ou un mélange des deux types de poloxamer. Le poloxamer permet, de part ses propriété physicochimiques comparables à la lipocaline, d'obtenir les propriétés d'abaissement de la tension de surface de l'eau de sorte à faciliter l'étalement de celle-ci sur la peau. De plus le poloxamer permet de stabiliser le film de lipide recouvrant le film d'eau.

Par « glycoprotéine » on entend un composé organique contenant à la fois une protéine et un carbohydrate ou chaîne oligosaccharidique liés par une liaison covalente. La chaîne oligosaccharidique se compose de 2 à 20 unités monosaccharidiques. Les sucres les plus communs des glycoprotéines sont le β-D-galactose, le α-D-mannose et le β-N-acétylgalactosamine, le α-L-fucose et les acides sialiques. Ces chaînes oligosaccharidiques sont soit branchées latéralement à la protéine soit occupent une position terminale sur la protéine. Ces glycoprotéines peuvent être issues de la famille des sérum-protéines, telles que les immunoglobulines, et les glycoprotéines sécrétoires ou mucines, produites par les « goblet cells » tant au niveau gastrointestinal, tractus respiratoire, salivaire, oculaire que sudorale.

On utilise préférentiellement dans l'association, objet de l'invention, les mucines. Toutefois pour des raisons de compatibilité avec la peau et notamment du fait de la réglementation en vigueur (problèmes dus à l'origine animale des glycoprotéines, par exemple dans le cas de la mucine Type II de porc fournie par Sigma), on peut avantageusement utiliser des glycoprotéines de type mucine d'origine végétale, bactérienne ou mycosique offrant des propriétés physicochimiques similaires.

L'acide chondroïtine sulfurique(ACS) peut se présenter sous trois formes A, B et C : 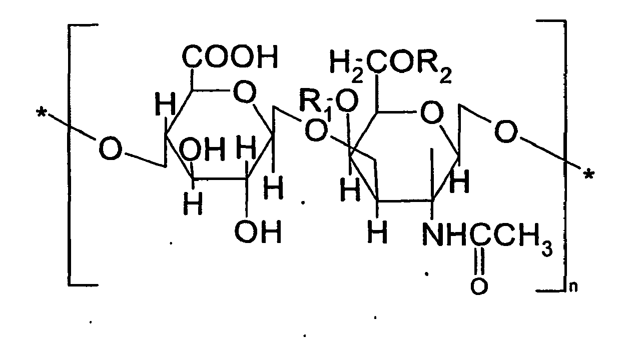 Acide chondroïtine sulfurique de forme A : R₁ = SO₃H,
R₂ = H (« chondroïtine 4-sulfate »).
Acide chondroïtine sulfurique de forme C : R₁ = H, R₂ = SO₃H (« chondroïtine 6-sulfate »).
Acide chondroïtine sulfurique de forme B : R₁ = SO₃H, R₂ = H, C-5' epimer (« β-heparin »)
Toutes ces formes d'acide chondroïtine sulfurique conviennent dans le cadre de la présente invention
On préfère l'ACS constituant du tissu cartilagineux et constitué d'un mélange des chondroïtines -4 et -6 sulfate (type A et C). On préfère tout particulièrement l'hydrolysat d'acide chondroïtine sulfurique marin et encore plus particulièrement celui de poids moléculaire compris entre 13 et 23 kDa. L'hydrolysat d'ACS obtenu par hydrolyse acide d'ACS natif d'origine marine peut être fourni par Rovafarm Argentina SA.

Dans le cas d'une association poloxamer et acide chondroïtine sulfurique, pour une valeur de 1 poloxamer en poids, la valeur en poids préférée en acide chondroïtine sulfurique varie de 1 à 3, de préférence varie de 1,6 à 2,4, de manière encore préférée varie de 1,8 à 2,2, la valeur préférée étant de 2.

Le ratio préféré est identique lorsque l'association selon la présente invention comporte des glycoprotéines, ou un mélange glycoprotéine/acide chondroïtine sulfurique, au lieu de l'acide chondroïtine sulfurique.

L'association selon la présente invention présente les caractéristiques d'un abaissement de la tension de surface de l'eau a une valeur comprise entre 37 et 44 mN/m et une tension interfaciale comprise entre 12 et 17 mN/m.
Ainsi l'association des deux polymères hydrosolubles selon l'invention présente l'avantage, d'abaisser la tension de surface, ce qui permet de structurer l'eau comme dans le cas du film oculaire, notamment en favorisant un meilleur étalement de l'eau sur une surface solide. L'étalement de l'eau sur une surface solide peut se mesurer par l'angle de contact.

L'association selon l'invention présente en outre l'avantage d'assurer la stabilisation du film de lipides grâce à la présence notamment du copolymère séquencé (le poloxamer) ayant une structure lipophile et hydrophile, associé à un abaissement de la tension interfaciale comprise entre 12 et 17 mN/m.

L'association selon la présente invention est tout particulièrement appropriée comme actif dans une composition cosmétique pour améliorer l'hydratation cutanée et/ou traiter la sécheresse cutanée.

Ainsi, la présente invention a également pour objet une composition cosmétique, caractérisée en ce qu'elle comprend une association telle que décrit ci-dessus ainsi qu'au moins un excipient cosmétiquement acceptable.

L'excipient cosmétiquement acceptable peut être tout excipient parmi ceux connus de l'homme de l'art en vue d'obtenir une composition pour l'application topique sous forme de crème, d'une lotion, d'un gel, d'une pommade, d'une émulsion, d'une microémulsion, d'un spray etc.

La composition selon l'invention peut en particulier contenir d'autres additifs et aides à la formulation, tels que des émulsionnants, des agents de texture (gélifiant, des stabilisants, des colorants, des parfums et des conservateurs).

D'autre actifs peuvent être inclus dans ces formulations, tels que des composés utilisées classiquement dans l'hydratation comme la glycérine, l'urée ou des agents du NMF (acides aminés, acide pyrrolidone carboxylique) ainsi que des composés anti-radicalaires (vitamine E ou dérivés) et des actifs anti-âges (dérivés rétinoïques) ou des filtres solaires (minéraux ou organiques).

Avantageusement, la composition objet de l'invention comprend au moins 3 phases :
(A) une association telle que décrite ci-dessus,
(B) de l'eau et,
(C) une phase grasse composée par des lipides sébacés ou par des lipides épidermiques ou bien par des lipides couramment utilisés en cosmétique.

L'eau utilisée pour la phase aqueuse (B) peut être une eau distillée ou une eau thermale ayant des propriétés dermo-cosmétique, telle que l'eau d'Avène.

Les lipides sébacés ou épidermiques peuvent être choisis parmi les céramides, le cholestérol et les acides gras. A titre de lipides de la phase (C) de la composition on peut encore utiliser des lipides d'origine sébacée tels que les triglycérides, le squalène ou les cires. Les lipides couramment utilisés en cosmétique possèdent typiquement une action émolliente ou occlusive. Parmi les lipides possédant une action émolliente on peut notamment citer les esters à longue chaîne tels que cétyloléate ou les esters de glycérol parmi lesquels le glycéryl myristate. Parmi les lipides possédant une action occlusive on peut citer l'huile de paraffine, l'huile de vaseline, la cire de jojoba, les triglycérides.
La phase (C) peut enfin être composée d'une association de lipides appartenant aux différentes familles de lipides décrites ci-dessus.

Dans la phase (A), l'association selon l'invention est présente selon une proportion comprise entre 0,01- 20 % et préférentiellement entre 0,1 et 1 % en poids par rapport au poids total de la composition; la concentration en acide chondroïtine sulfurique et/ou en glycoprotéine est comprise entre 0,01- 5% et préférentiellement entre 0,1 et 1 % en poids par rapport au poids total de la composition et la concentration en poloxamer est comprise entre 0,001- 20 % et préférentiellement entre 0,05 et 1 % en poids par rapport au poids total de la composition.

Dans la phase (B), l'eau est comprise entre 20 et 99% en poids par rapport au poids total de la composition.

Dans la phase (C), les lipides sont compris entre 1% et 50% en poids par rapport au poids total de la composition.

La composition cosmétique selon la présente invention permet ainsi une meilleure hydratation de la peau selon un processus dynamique :
- le premier aspect de l'action de la composition se situe au niveau d'une meilleure biodisponibilité de la phase aqueuse, consécutive à un étalement, accru (surface de contact importante). On pourrait penser que cette surface de contact accrue conduit aussi à une plus grande évaporation. Toutefois celle-ci est ralentie par la présence de la phase lipidique, stabilisée par l'association de polymères.

Cette association permet, en outre, de potentialiser l'effet hydratant des molécules classiquement utilisé pour hydrater le stratum corneum, telle que la glycérine.
- Selon un deuxième aspect, la pénétration de l'eau dans le stratum corneum conduit à une hydratation des couches inférieures ; les lipides viennent alors s'insérer dans les espaces intercellulaires afin de renforcer les capacités de la fonction barrière, qui est de freiner la perte en eau. La composition cosmétique permet donc de maintenir une hydratation cutanée optimum.

Ainsi, la présente invention a également pour objet un procédé de traitement cosmétique pour améliorer l'hydratation cutanée caractérisé en ce que l'on applique sur la peau une composition cosmétique telle que définie ci-dessus.

On a par ailleurs constaté que l'association selon la présente invention peut être utilisée dans une composition oculaire dans le cadre du traitement du syndrome de l'oeil sec.
Elle présente l'avantage de conférer à la composition oculaire une bonne tolérance.

La présente invention a donc également pour objet une composition oculaire contenant l'association telle que décrite précédemment et l'utilisation de ladite association pour la préparation d'une composition oculaire destinée au traitement du syndrome de l'oeil sec.

Avantageusement, une telle composition ophtalmique, objet de l'invention, comprend au moins deux phases :
a) une association objet de l'invention,
b) de l'eau et des sels dissous (solution isotonique ou hypotonique), un conservateur, ainsi que des excipients utilisés en applications oculaires tels que le sorbitol ou l'édétate de sodium.

Une troisième phase c) peut être introduite dans la composition oculaire. Elle peut être composée de lipides mimant les lipides oculaires, associant des phospholipides tels que la diodéyl-phosphatidyl choline, des cires telles que des esters à longues chaînes et des triglycérides tels que la tristéarine.

La présente invention a enfin pour objet une composition pour le traitement de la sécheresse bucco-gingivale et une composition pour la sécheresse vaginale contenant l'association objet de l'invention ainsi que l'utilisation de l'association, objet de l'invention, pour la préparation de ces compositions.

Les exemples suivants sont destinés à illustrer la présente invention et ne doivent en aucun cas être interprétés comme pouvant en limiter la portée.

### Exemple 1 : résultats de tension de surface, de tension interfaciale et de valeurs d'angle de contact d'une association selon l'invention

**Tableau I : valeurs de tension de surface et de tension interfaciale de l'eau en présence de mucines ou d'acide chondroïtine sulfurique (ACS) associé au poloxamer**

| | **Mucin II¹** | | | **ACS marin ²** | | |
|---|---|---|---|---|---|---|
| | tension de surface (mN/m) | Tension Interfaciale Myritol (mN/m) | Tension Interfaciale H.Paraffine (mN/m) | Tension de surface (mN/m) | Tension Interfaciale Myritol (mN/m) | Tension Interfaciale H.Paraffine (mN/m) |
| seul(0,5%) | 40,65 | 12,45 | 25,38 | 54,04 | 20,75 | 31,83 |
| avec Poloxamer 188 ³ (0,5%) | 43,20 | 13,17 | 16,83 | 41,65 | 12,83 | 15,86 |
| avec Poloxamer 407 ⁴ (0,5%) | 38,56 | 6,27 | 9,83 | 37,14 | 5,20 | 9,04 |

| | | | | | | |
|---|---|---|---|---|---|---|
| **Physicochimie de l'oeil** - Tension de surface « larmes » 40 - 45 mN/m - Tension interfaciale « larmes - film de lipides » environ 15 mN/m | | | | | | |

La tension de surface est une grandeur physique mesurée entre un liquide et l'air. Cette grandeur nous renseigne sur la présence de composés absorbés sur l'interface liquide-air. Ainsi, on observe une baisse de tension de surface lors de l'ajout de tensio-actif dans l'eau. De manière identique, la mesure de la tension interfaciale renseigne sur l'absorption préférentielle sur l'interface « eau-huile ». Ainsi l'huile peut être de l'huile de paraffine (totalement apolaire) ou une huile plus polaire telle que le myritol.

Afin de répondre aux critères physicochimiques de l'oeil, la tension de surface est de l'ordre de 40 mN/m et la tension interfaciale « eau-huile » est de l'ordre de 15 mN/m.

Ainsi dans le tableau I, on montre que l'association de la mucine II et du poloxamer 188 ou 407 ou bien l'association de l'ACS et du poloxamer 188 ou 407, permet de répondre aux critères de l'oeil, tant en tension de surface que pour la tension interfaciale que ce soit vis-à-vis de l'huile de paraffine ou du myritol.

**Tableau II : valeurs d'angle de contact de l'eau en présence de mucines ou ACS associé au poloxamer sur une surface très hydrophobe (parafilm) et plus hydrophile (verre).**

| | **Mucin II ¹** | | **ACS marin ²** | |
|---|---|---|---|---|
| | Verre | Parafilm | Verre | Parafilm |
| seul | 52,70 | 88,90 | 62,80 | 89,70 |
| avec Poloxamer 188 ³ (0,5%) | 47,60 | 78,80 | 41,10 | 83,30 |
| avec Poloxamer 407 ⁴ (0,5%) | 39,70 | 73,70 | 41,80 | 70,90 |

| | | | | |
|---|---|---|---|---|
| ¹ commercialisé par la société SIGMA ² commercialisé par la société Rovafarm Argentine SA ³ commercialisé par la société BASF ⁴ commercialisé par la société BASF | | | | |

Les mesures d'angles de contact réalisées montrent un meilleur étalement de l'eau en présence de l'association sur deux supports solides, l'un très hydrophobe, le parafilm ou sur le verre, plus hydrophile. Ceci confirme le rôle d'agent mouillant apporté par l'association selon l'invention.

### Exemple 2 : formulation, émulsion

| | En gramme |
|---|---|
| Hyaluronate | 0,01 |
| Extrait lipidique | 0,1 |
| Beta sitostérol | 0,1 |
| Poloxamer 188 | 0,1 |
| Acide chondroïtine sulfurique | 0,2 |
| Glycérine végétal | 3 |
| Butane diol 1-3 | 2 |
| Huile de carthame | 5 |
| Triglycérides caprique caprylique | 5 |
| Distéarate de succynil | 5 |
| Décamethyl cyclo | 5 |
| Synthalen K | 0,58 |
| Hydroxyde de sodium | 0,15 |
| « Micropearl » | 2 |
| Phénoxyéthanol | 0,35 |
| Chlorphenesin | 0,25 |
| Acide benzoïque | 0,1 |
| Edetate de sodium | 0,2 |
| Parfum "rich cream" | 0,15 |
| Eau d'Avène | 70,9 |

### Exemple 3 : solution ophtalmique

| | En gramme |
|---|---|
| Poloxamer 188 | 0,2 |
| ACS | 0,4 |
| Acide benzoique | 0,1 |
| Hydroxyde de Na | 0,5 |
| Edetate de Na | 0,2 |
| Cétrimide | 0,05 |
| Sorbitol | 3,0 |
| Eau | qsp 100g |

### Exemple 4 : solution bucco-gingivale

| | |
|---|---|
| Poloxamer 188 | 0,1 |
| ACS | 0,2 |
| Hydroxyde de Na | 0,5 |
| Edetate de Na | 0,2 |
| Cétrimide | 0,05 |
| Glycérol | 5,0 |
| Carbopol | 0,2 |
| Eau | qsp 100g |

## Revendications

1. Association d'un poloxamer avec l'acide chondroïtine sulfurique, **caractérisée en ce que** pour une valeur de 1 poloxamer en poids, la valeur en poids en acide chondroïtine sulfurique varie de 1 à 3, de préférence de 1,6 à 2, 4, de manière entre plus préférée de 1,8 à 2,2, plus préférentiellement encore égale à 2.

2. Association selon la revendication 1, **caractérisée en ce que** le poloxamer est un copolymère séquencé polyoxyethylène-polyoxypropylène-polyoxyethylène (POE-POP-POE) de formule générale : 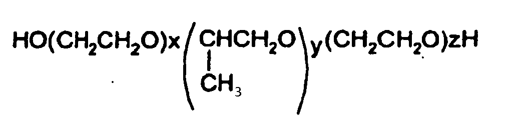 dans laquelle,
la valeur moyenne de x est comprise entre 2 et 128, la valeur moyenne de y est comprise entre 16 et 67 et la valeur moyenne de z est comprise entre 2 et 128.

3. Association selon la revendication 1 ou 2, **caractérisée en ce que** le poloxamer est le poloxamer 188 ou le poloxamer 407 ou un mélange de ces deux types de poloxamer.

4. Association selon l'une quelconque des revendications 1 à 3, **caractérisée en ce que** l'acide chondroïtine sulfurique est le mélange d'acides chondroïtine de type A et C extraits de tissus cartilagineux.

5. Association selon la revendication 4, **caractérisée en ce que** l'acide chondroïtine sulfurique est un hydrolysat marin d'acide chondroïtine sulfurique.

6. Association selon la revendication 1, **caractérisée en ce qu'**elle contient en plus une glycoprotéine.

7. Association selon la revendication 6, **caractérisée en ce que** la glycoprotéine comporte dans la chaîne oligosaccharidique 2 à 20 unités monosaccharidiques choisies parmi le β-galactose, le α-D-mannose et le β-N-acétylgalactosamine, le α-L-fucose et les acides sialiques.

8. Association selon la revendication 6, **caractérisée en ce que** la glycoprotéine est une mucine.

9. Association selon la revendication 8, **caractérisée en ce que** la mucine est d'origine végétale, bactérienne ou mycosique.

10. Association selon l'une quelconque des revendications 1 à 9 **caractérisée en ce qu'**elle comprend un poloxamer tel que défini dans l'une des revendications 2 ou 3 et l'acide chondroïtine tel que défini dans les revendications 4 ou 5.

11. Composition cosmétique, **caractérisée en ce qu'**elle comprend une association selon l'une quelconque des revendications 1 à 10.

12. Composition cosmétique selon la revendication 11, **caractérisée en ce qu'**elle comprend au moins 3 phases :
(A) une association selon l'une quelconque des revendications 1 à 10,
(B) de l'eau et,
(C) une phase grasse composée par des lipides sébacés ou par des lipides épidermiques, par des lipides à action émolliente ou occlusive ou bien par une association de ces lipides.

13. Composition cosmétique selon la revendication 12, **caractérisée en ce que** les lipides sébacés ou épidermiques sont choisis parmi les céramides, le cholestérol, les acides gras, les triglycérides, le squalène et les cires.

14. Composition cosmétique selon la revendication 12, **caractérisée en ce que** les lipides à action émolliente ou occlusive sont choisis parmi l'huile de paraffine l'huile de vaseline ou l'huile de jojoba.

15. Composition cosmétique selon l'un quelconque des revendications 12 à 14, **caractérisée en ce que**
- dans la phase (A) telle que définie à la revendication 13, l'association est présente selon une proportion comprise entre 0,01 et 12 % en poids par rapport au poids total de la composition,
- dans la phase (B) telle que définie à la revendication 13, l'eau est comprise entre 20 et 99% en poids par rapport au poids total de la composition,
dans la phase (C) telle que définie à la revendication 13, les lipides sont compris entre 1 et 50% en poids par rapport au poids total de la composition.

16. Utilisation de l'association selon l'un quelconque des revendications 1 à 10 pour la préparation d'une composition cosmétique destinée à améliorer l'hydratation cutanée et/ou traiter la sécheresse cutanée.

17. Procédé de traitement cosmétique pour améliorer l'hydratation cutanée
**caractérisé en ce que** l'on applique sur la peau une composition cosmétique selon l'une quelconque des revendications 11 à 16.

18. Composition oculaire contenant l'association telle que définie aux revendications 1 à 10.

19. Composition oculaire selon la revendication 18 comprenant en outre de l'eau et des sels dissous.

20. Composition oculaire selon la revendication 19 comprenant en outre un conservateur ainsi que du sorbitol ou de l'édétate de sodium.

21. Composition oculaire selon la revendication 19 ou 20 comprenant en outre des lipides associant des phospholipides, des cires et des triglycérides.

22. Utilisation de l'association telle que définie dans la revendication 1 à 10 pour la préparation d'une composition oculaire destinée au traitement du syndrome de l'oeil sec.

23. Composition pour le traitement de la sécheresse bucco-gingivale contenant l'association telle que définie aux revendications 1 à 10.

24. Composition pour le traitement de la sécheresse vaginale contenant l'association telle que définie aux revendications 1 à 10.

25. Utilisation de l'association telle que définie dans les revendications 1 à 10 pour la préparation d'une composition destinée au traitement de la sécheresses bucco-gingivale ou vaginale.

## Claims

1. Association of a poloxamer with sulphuric chondroitin acid, **characterised in that** for a value of 1 poloxamer by weight, the value by weight of sulphuric chondroitin acid varies from 1 to 3, preferably from 1.6 to 2.4, more preferably from 1.8 to 2.2, and is yet more preferably equal to 2.

2. Association according to claim 1, **characterised in that** the poloxamer is a polyoxyethylene - polyoxypropylene - polyoxyethylene (POE - POP - POE) sequenced copolymer with the following general formula: 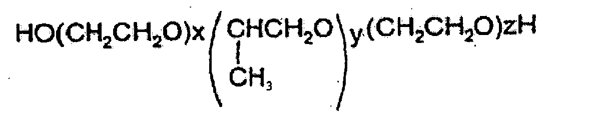 in which,
the average value of x is between 2 and 128, the average value of y is between 16 and 67, and the average value of z is between 2 and 128.

3. Association according to claim 1 or 2, **characterised in that** the poloxamer is poloxamer 188 or poloxamer 407 or a mixture of these two types of poloxamer.

4. Association according to any of claims 1 to 3, **characterised in that** sulphuric chondroitin acid is the mixture of chondroitin acids of types A and C extracted from cartilaginous tissues.

5. Association according to claim 4, **characterised in that** sulphuric chondroitin acid is a marine sulphuric chondroitin acid hydrolysate.

6. Association according to claim 1, **characterised in that** it further contains a glycoprotein.

7. Association according to claim 6, **characterised in that** the glycoprotein comprises in the oligosaccharide chain 2 to 20 monosaccharide units, chosen from among β-galactose, α-D-mannose and β-N-acetylgalactosamine, α-L-fucose and sialic acids.

8. Association according to claim 6, **characterised in that** the glycoprotein is a mucin.

9. Association according to claim 8, **characterised in that** the mucin is of vegetable, bacterial or mycotic origin.

10. Association according to any of claims 1 to 9, **characterised in that** it includes a poloxamer as defined in either claim 2 or 3 and chondroitin acid as defined in claim 4 or 5.

11. Cosmetic composition, **characterised in that** it includes an association according to any of claims 1 to 10.

12. Cosmetic composition according to claim 11, **characterised in that** it includes at least three phases:
(A) an association according to any of claims 1 to 10,
(B) water and,
(C) a fatty phase composed of sebaceous lipids or epidermic lipids, lipids with an emollient or occlusive action, or else an association of these lipids.

13. Cosmetic composition according to claim 12, **characterised in that** the sebaceous or epidermic lipids are chosen from among ceramides, cholesterol, fatty acids, triglycerides, squalene and waxes.

14. Cosmetic composition according to claim 12, **characterised in that** lipids with an emollient or occlusive action are chosen from among paraffin oil, vaseline oil or jojoba oil.

15. Cosmetic composition according to any of claims 12 to 14, **characterised in that**
- in phase (A) as defined in claim 13, the association is present in a proportion of between 0.01 and 12% by weight of the total weight of the composition,
- in phase (B) as defined in claim 13, the water is between 20 and 99% by weight of the total weight of the composition,
- in phase (C) as defined in claim 13, the lipids are between 1 and 50% by weight of the total weight of the composition.

16. Use of the association according to any of claims 1 to 10 for the preparation of a cosmetic composition intended to improve skin moisturisation and / or treat skin dryness.

17. Cosmetic treatment method to improve skin moisturisation, **characterised in that** a cosmetic composition according to any of claims 11 to 16 is applied to the skin.

18. Ocular composition containing the association as defined in claims 1 to 10.

19. Ocular composition according to claim 18, further including water and dissolved salts.

20. Ocular composition according to claim 19, further including a preserving agent as well as sorbitol or sodium edetate.

21. Ocular composition according to claim 19 or 20, further including lipids associating phospholipids, waxes and triglycerides.

22. Use of the association as defined in claims 1 to 10, for the preparation of an ocular composition intended for the treatment of dry eye syndrome.

23. Composition for the treatment of bucco-gingival dryness containing the association as defined in claims 1 to 10.

24. Composition for the treatment of vaginal dryness containing the association as defined in claims 1 to 10.

25. Use of the association as defined in claims 1 to 10, for the preparation of a composition intended for the treatment of bucco-gingival or vaginal dryness.

## Patentansprüche

1. Kombination eines Poloxamers mit Chondroitinschwefelsäure, **dadurch gekennzeichnet, dass** für einen Gewichtswert von Poloxamer von 1 der Gewichtswert an Chondroitinschwefelsäure von 1 bis 3, vorzugsweise von 1,6 bis 2,4, noch mehr bevorzugt von 1,8 bis 2,2 variiert, noch mehr bevorzugt gleich 2 ist.

2. Kombination nach Anspruch 1, **dadurch gekennzeichnet, dass** das Poloxamer ein Copolymer mit der Sequenz Polyoxyethylen-Polyoxypropylen-Polyoxyethylen (POE-POP-POE) der allgemeinen Formel: 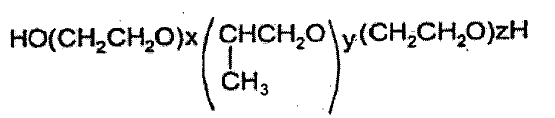 ist, in welcher der Mittelwert von x zwischen 2 und 128 eingeschlossen liegt, der Mittelwert von y zwischen 16 und 67 eingeschlossen liegt und der Mittelwert von z zwischen 2 und 128 eingeschlossen liegt.

3. Kombination nach Anspruch 1 oder 2, **dadurch gekennzeichnet, dass** das Poloxamer Poloxamer 188 oder Poloxamer 407 oder eine Mischung von diesen beiden Poloxamerarten ist.

4. Kombination nach einem der Ansprüche 1 bis 3, **dadurch gekennzeichnet, dass** die Chondroitinschwefelsäure die Mischung von Chondroitinsäuren vom Typ A und C, die aus Knorpelgeweben extrahiert werden, ist.

5. Kombination nach Anspruch 4, **dadurch gekennzeichnet, dass** die Chondroitinschwefelsäure ein marines Hydrolysat von Chondroitinschwefelsäure ist.

6. Kombination nach Anspruch 1, **dadurch gekennzeichnet, dass** sie außerdem ein Glycoprotein enthält.

7. Kombination nach Anspruch 6, **dadurch gekennzeichnet, dass** das Glycoprotein in der Oligosaccharidkette 2 bis 20 Monosaccharid-Einheiten, ausgewählt unter β-Galactose, α-D-Mannose und β-N-Acetylgalactosamin, α-L-Fucose und den Sialinsäuren, umfasst.

8. Kombination nach Anspruch 6, **dadurch gekennzeichnet, dass** das Glycoprotein ein Mukoid ist.

9. Kombination nach Anspruch 8, **dadurch gekennzeichnet, dass** das Mukoid von pflanzlichem, bakteriellem oder pilzlichem Ursprung ist.

10. Kombination nach einem der Ansprüche 1 bis 9, **dadurch gekennzeichnet, dass** sie ein Poloxamer, wie in einem der Ansprüche 2 oder 3 definiert, und Chondroitinsäure, wie in den Ansprüchen 4 oder 5 definiert, umfasst.

11. Kosmetische Zusammensetzung, **dadurch gekennzeichnet, dass** sie eine Kombination nach einem der Ansprüche 1 bis 10 umfasst.

12. Kosmetische Zusammensetzung nach Anspruch 11, **dadurch gekennzeichnet, dass** sie mindestens 3 Phasen umfasst:
(A) eine Kombination nach einem der Ansprüche 1 bis 10,
(B) Wasser und
(C) eine fette Phase, welche aus talgartigen Lipiden oder epidermalen Lipiden, aus Lipiden mit weichmachender oder okklusiver Wirkung oder ebenso aus einer Kombination von diesen Lipiden gebildet wird.

13. Kosmetische Zusammensetzung nach Anspruch 12, **dadurch gekennzeichnet, dass** die talgartigen oder epidermalen Lipide unter den Ceramiden, Cholesterol, den Fettsäuren, den Triglyceriden, Squalen und den Wachsen ausgewählt sind.

14. Kosmetische Zusammensetzung nach Anspruch 12, **dadurch gekennzeichnet, dass** die Lipide mit weichmachender oder okklusiver Wirkung unter Paraffinöl, Vaselinöl oder Jojobaöl ausgewählt sind.

15. Kosmetische Zusammensetzung nach einem der Ansprüche 12 bis 14, **dadurch gekennzeichnet, dass**
- in der Phase (A), wie sie in Anspruch 13 definiert ist, die Kombination gemäß einem Anteil zwischen 0,01 und 12 Gew.-% eingeschlossen bezogen auf das Gesamtgewicht der Zusammensetzung vorhanden ist,
- in der Phase (B), wie sie in Anspruch 13 definiert ist, das Wasser zwischen 20 und 99 Gew.-% eingeschlossen bezogen auf das Gesamtgewicht der Zusammensetzung ausmacht;
- in der Phase (C), wie sie in Anspruch 13 definiert ist, die Lipide zwischen 1 und 50 Gew.-% eingeschlossen bezogen auf das Gesamtgewicht der Zusammensetzung ausmachen.

16. Verwendung der Kombination nach einem der Ansprüche 1 bis 10 für die Herstellung einer kosmetischen Zusammensetzung, die dazu bestimmt ist, die Hydratisierung der Haut zu verbessern und/oder Hauttrockenheit zu behandeln.

17. Kosmetisches Behandlungsverfahren, um die Hydratisierung der Haut zu verbessern, **dadurch gekennzeichnet, dass** man auf die Haut eine kosmetische Zusammensetzung nach einem der Ansprüche 11 bis 16 aufträgt.

18. Okulare Zusammensetzung, welche die Kombination, wie sie in den Ansprüchen 1 bis 10 definiert ist, enthält.

19. Okulare Zusammensetzung nach Anspruch 18, welche außerdem Wasser und gelöste Salze umfasst.

20. Okulare Zusammensetzung nach Anspruch 19, welche außerdem ein Konservierungsmittel wie auch Sorbitol oder Natriumedetat umfasst.

21. Okulare Zusammensetzung nach Anspruch 19 oder 20, welche außerdem Lipide, welche Phospholipide verketten, Wachse und Triglyceride umfasst.

22. Verwendung der Kombination, wie sie in Anspruch 1 bis 10 definiert ist, für die Herstellung einer okularen Zusammensetzung, die für die Behandlung des Syndroms des trockenen Auges bestimmt ist.

23. Zusammensetzung für die Behandlung von bukkogingivaler Trockenheit, welche die Kombination, wie sie in den Ansprüchen 1 bis 10 definiert ist, enthält.

24. Zusammensetzung für die Behandlung von vaginaler Trockenheit, welche die Kombination, wie sie in den Ansprüchen 1 bis 10 definiert ist, enthält.

25. Verwendung der Kombination, wie sie in den Ansprüchen 1 bis 10 definiert ist, für die Herstellung einer Zusammensetzung, die für die Behandlung von bukkogingivaler oder vaginaler Trockenheit bestimmt ist.
